(19) Europäisches Patentamt
European Patent Office
Office européen des brevets

(11) Publication number: **0 092 121**
**B1**

(12) **EUROPEAN PATENT SPECIFICATION**

(45) Date of publication of patent specification: **16.12.87**

(51) Int. Cl.$^4$: **A 61 K 31/685**

(21) Application number: **83103437.6**

(22) Date of filing: **08.04.83**

(54) **Methods and compositions for treating gastro-intestinal ulcer diesease.**

(30) Priority: **12.04.82 US 367451**

(43) Date of publication of application:
**26.10.83 Bulletin 83/43**

(45) Publication of the grant of the patent:
**16.12.87 Bulletin 87/51**

(84) Designated Contracting States:
**CH DE FR GB LI SE**

(56) References cited:
**FR-A-2 445 146**
**FR-M- 5 060**

**CHEMICAL ABSTRACTS, vol.92, no.3, January 21, 1980, abstract no.15574p, page 62, Columbus, Ohio, US; M.IKEGAMI et al.: "Restoration of lung pressure-volumne characteristics with various phospholipids"**

(73) Proprietor: **THE UNIVERSITY OF TEXAS SYSTEM Board of Regents**
**201 West 7th Street**
**Austin, Texas 78701 (US)**

(72) Inventor: **Lichtenberger, Lenard M.**
**5427 Darnell**
**Houston Texas 77096 (US)**
Inventor: **Hills, Brian A.**
**25 Wilderness Trail**
**Friendswood Texas 77546 (US)**

(74) Representative: **Howden, Christopher Andrew**
**FORRESTER & BOEHMERT Widenmayerstrasse 4/I**
**D-8000 München 22 (DE)**

**EP 0 092 121 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to the use of amphoteric phospholipid surfactant(s) for the manufacture of a medicament, in a form suitable for internal administration for treating gastro-intestinal ulcer disease, and to a composition for treating gastro-intestinal ulcer disease comprising amphoteric phospholipid surfactants in a form suitable for internal administration, wherein the surfactant is a mixture of phosphatidylcholine, phosphatidyl, ethanolamine, phosphatidylserine and sphingomyelin.

Gastro-intestinal ulcer disease, in particular, peptic ulcers, affect 5—15% of the United States population. Moreover, this disease is not restricted to the more industrialized Western culture; indeed gastric ulceration is even a more serious problem in the Orient. One ulcer disease, particularly worrisome to pediatricians, occurs in the premature infants. This condition, known as necrotizing enterocolitis, affects 10—15% of newborns having a birth weight of under 1.5 kg and results in severe intestinal ulceration, which frequently requires surgery. The etiology of this condition, like that of peptic ulcer disease, is not understood but it has been postulated that the primary defect lies in an abnormal mucosal defense mechanism against luminal damaging agents.

There are many anti-ulcer drugs currently on the market. Most of these drugs are directed to neutralizing or inhibiting gastric acid secretion. Notable of the anti-ulcer compositions are anticholinergics and anti-histamines both of which are indirect means of treatment, and result in a multitude of undesirable side effects in addition to blocking gastric acid secretion. This form of therapy is based on the tenet "no acid, no ulcer". Although it appears that ulcers will not form in the complete absence of gastric acid, it is generally recognized that not all ulcer patients exhibit enhanced gastric acid output. In fact, gastric ulcer patients as a group have abnormally low gastric acidity. Thus, it has been suggested that gastric acidity may only be an aggravating factor and not a primary cause of gastro-intestinal ulcerogenesis.

An alternative explanation of ulcer incipiency involves the belief that G.I. ulceration develops in individuals that have a defect in a putative "gastric mucosal barrier". This defect permits luminal damaging agents acid, enzymes, bile salts, bacteria) to penetrate the surface lining the thereafter promote ulcerogenesis.

It is presently unclear how the normal gastro-intestinal (GI) epithelium protects itself from these insults. Indeed, the answer to this fundamental question has long been sought, since it certainly remains a paradox why the stomach does not digest itself while it is constantly bathed in an extremely acidic and proteolytic environment. Conversely, the clinically important question remains as to how and why the element of protection is removed or circumvented in ulcer disease and necrotizing enterocolitis. A great deal of research has been performed to answer these important questions. Investigators have postulated that the mucosa is protected by a putative "gastro-intestinal mucosal barrier" which prevents the back diffusion of hydrochloric acid and other potentially toxic agents from the lumen into the epithelium. Disruption of this mucosal barrier, results in the development of GI erosions. Although a wide variety of damaging agents such as aspirin, bile salts, hydrochloric acid and alcohol certainly will cause GI ulceration if present in high enough concentrations, it is generally believed that the primary cause of ulcer desease in a majority of patients is attributable to a natural defect in the "GI mucosal barrier".

Recent studies by the applicants have indicated that many of the phospholipids present in pulmonary fluid are also found along the length of the GI tract from the esophagus to the colon. These phospholipids appear to be concentrated on the mucosal surface of these tissues which separate the digestive and absorptive epithelium from the luminal contents. The functional importance of these compounds has been studied in greatest detail in the lung. It is now well recognized that pulmonary surfactants play a vital role in minimizing the surface forces at the level of the alveoli, allowing the alveoli to remain open throughout the respiratory cycle.

There is a certain amount of evidence that the surface properties of surfactants also play a role in reducing the movements of extracellular fluid from the blood into the extracellular space. Related to this property, one of the Applicants has recently demonstrated that surfactants, not unlike commercially available water repellents used to treat material surfaces, will make biological tissues non-wettable. This action provides the alveoli with a hydrophobic lining that will resist the penetration of water molecules across its surface.

In recent experiments culminating to the present invention, Applicants considered the possibility that a phospholipid coating of the GI mucosal epithelium may similarly provide that tissue with a hydrophobic surface coating. If so, this may explain why damaging agents, within the lumen, most of which are dissolved in aqueous solutions, fail to penetrate the mucosal epithelium. Accordingly, if the integrity of the barrier is indeed the primary defect in GI ulcerogenesis, then the remedy of choice is to administer drugs which would restore the competence of the surface barrier. The present invention seeks to develop such compositions and methods of treatment effective to repair and prevent GI ulcerative erosion.

Figure 1 is a schematic representation of how amphoteric phospholipids which become cationic at the pH (acidity) of the stomach will thus be adsorbed onto a negatively charged surface membrane to form a uniform hydrophobic layer. This hydrophobic layer should serve as a barrier to hydrogen ions and other damaging agents dissolved in aqueous solutions.

Figure 2 is a graphical illustration of data reflecting the effect of the presence —○— and absence —— of a phospholipid suspension on the mucosal to serosal $H^+$ flux across the canine gastric mucosae *in vitro*.

The pH of the nutrient compartment was continuously monitored after HCl (900 mM) was added to the mucosal compartment of an Ussing Chamber. Each point represents the mean of seven experiments.

FR—A—2445146 (and U.K. 2039738A) teaches the use of phospholipids in conjunction with non-steroidal anti-inflammatory substances so as to prevent the undesirable gastric side effects that often occur through the use of these drugs.

Chemical Abstracts, Vol. 92, Abstract 15574 p, page 62 discloses the use of various phospholipids to restore lung pressure-volume characteristics and FR—M—5060M discloses the use of phospholipids but in no way hints or suggests the treatment of gastro-intestinal ulcer disease therewith.

In accordance with the present invention there is provided the use of amphoteric phospholipid surfactant(s) for the manufacture of a medicament, in a form suitable for internal administration for treating gastro-intestinal ulcer disease. The present invention also provides a composition for treating gastro-intestinal ulcer disease comprising amphoteric phospholipid surfactants in a form suitable for internal administration, wherein the surfactant is a mixture of phosphatidylcholine, phosphatidylethanolamine, phosphatidylserine and sphingomyelin.

Generally in accordance with the present invention, a phospholipid compound either individually or in combination with other phospholipid compounds together in a pharmaceutically acceptable dosage form is administered perorally to a subject susceptible to gastro-intestinal ulceration. The composition of phospholipid surfactants is effective to coat the mucosal surface and thereby establish a hydrophobic barrier sufficient to deter acid flux through the mucosal-serosal membrane.

Further in accordance with this invention, an anti-ulcer pharmaceutical composition which includes both amphoteric and anionic phospholipid surfactants is provided which is effective for the treatment of gastro-intestinal ulceration. The inclusion of an anionic phospholipid surfactant to amphoteric phospholipid composition appears to enhance the rate of phospholipid deposition onto the mucosal surface.

The invention will be described in terms of preferred embodiments which represent the best mode known to the Applicants at the time of this application.

In accordance with such preferred embodiments of this invention, phospholipid surfactant compositions are provided as an effective treatment of gastro-intestinal ulcer disease. Gastro-intestinal disease includes for example, but not limited to, peptic ulcers, gastric ulcers, duodenum ulcers, jejunal ulcers, esophageal ulcers and necrotizing enterocolitis.

According to the invention, a composition containing an effective amount of phospholipid surfactant is administered to a subject affected with or prone to gastro-intestinal ulcers, which composition is effective to mitigate and prevent subsequent gastro-intestinal ulceration. The amphoteric phospholipid surfactants of the invention encompass long chain aliphatic disubstituted phosphoglycerol type compounds having a phosphoro-substitution exhibiting a terminal functional group capable of forming a cationic charge. The pharmaceutically active ingredient of the compositions of the present invention include as a general class the phospholipids: lecithins, cephalins, sphingomyelins, phosphatidyl glycerols, phosphatidylserines and phosphatidylinositoles.

The following formulas are representative but not exclusive of the general structures of phospholipids which are encompassed by the compositions and methods of the present invention:

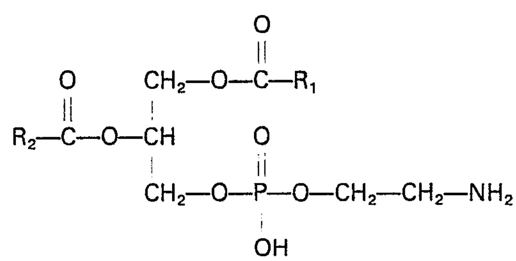

A. phosphatidylethanolamine (cephalin);

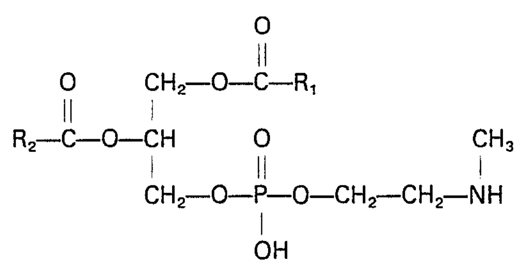

B. phosphatidylmonomethylethanolamine;

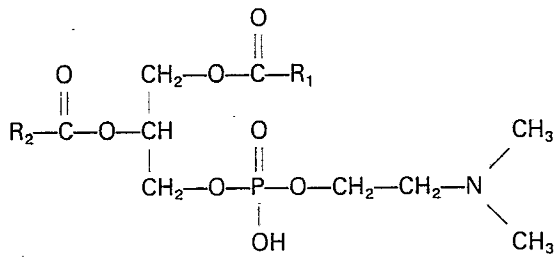

C. phosphatidyldimethylethanolamine; and

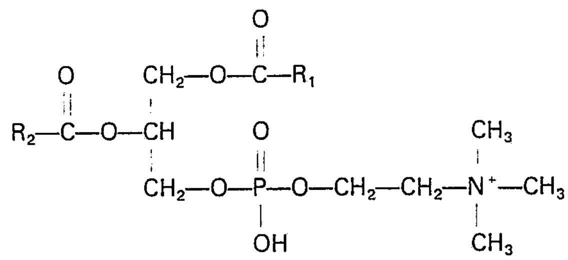

D. phosphatidylcholine;

wherein $R_1$ and $R_2$ are each independently an aliphatic group, saturated or unsaturated, having from 9 to 21 carbon atoms, suitably 13 to 21 carbon atoms, and preferably 13 to 17 carbon atoms.

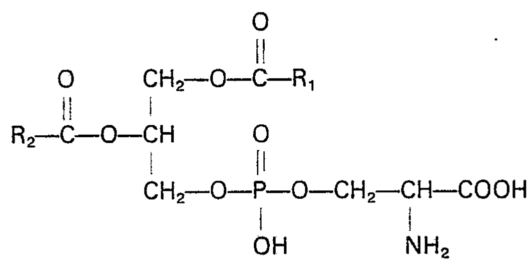

E. phosphatidylserine

Further in accordance with the compositions of the present invention, anionic phospholipids having the general formula

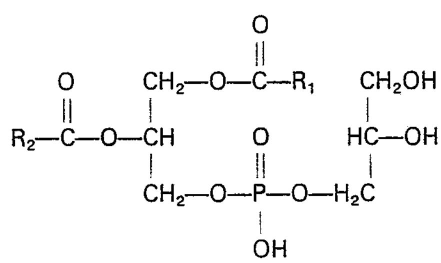

F. phosphatidylglycerol

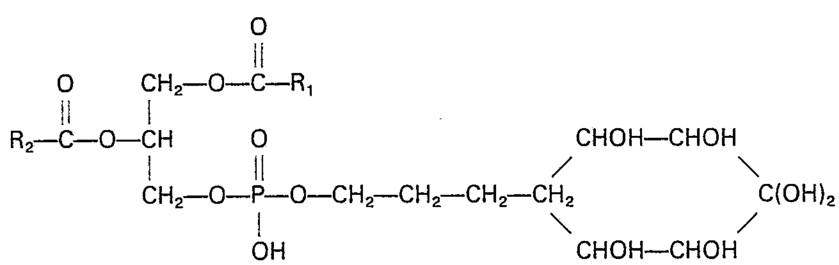

G. phosphatidylinositols

wherein $R_1$ and $R_2$ are each independently an aliphatic group, saturated or unsaturated, having from 9 to 21 carbon atoms, suitably 13 to 21 carbon atoms, and preferably 13 to 17 carbon atoms, can be combined with one or more of the above described amphoteric phospholipid surfactants effective to accelerate and promote the surface deposition and anti-ulcer activity of the phospholipids.

In particular, the specific compounds, dipalmitoyl lecithin (DPL); dipalmitoyl phosphatidyl ethanolamine (DPPE); sphingomyelin (Sphg); and dipalmitoyl phosphatidyl serine (DPPS) are preferred amphoteric phospholipid surfactants useful in the practice of this invention. Dipalmitoyl phosphatidyl glycerol (DPPG) and dipalimitoyl phosphatidyl inositol (DPPI) are the preferred anionic surfactants to be used in combination with one or more of the amphoteric phospholipids.

Under acidic conditions, as specifically encountered in the gastric environment, the above described amphoteric phospholipid surfactants typically accept a hydrogen ion at the amino terminal end thereby exhibiting a positive or cationic charge, hence they are effectively cationic.

The phospholipids which are the subject of this invention are naturally occurring substances extractable from plant and animal sources. Moreover many of the phospholipids detailed above can be synthesized by various known processes. Furthermore, many of the phospholipids are commercially available, as they are starting materials for a wide class of soaps, pharmaceutical preparations, and biochemical research materials.

The present invention is directed to the use of these compounds for the manufacture of a medicament for the treatment and prevention of gastro-intestinal ulceration, the treatment involving internally administering to a subject one or more phospholipids in a pharmaceutically effective amount. The preferred route is by mouth, but other routes such as intranasal spray can also be employed. The routes of administration are however limited to those routes which permit deposition of a sufficient surface coating of the phospholipid surfactant on an ulcer prone or affected mucosal layer.

For oral administration, the compounds can be administered in substantially pure, undiluted form; or in various pharmaceutical dosage forms such as capsules, liposome carriers, aerosol sprays, dispersions, aqueous suspensions and solutions.

While wishing not to be limited to the following theory, Applicants' invention is based on the principle that surfactants, which carry a positive terminal charge will be attracted to the negatively charged surface membrance of the GI tract. These molecules will in turn orient in such a way (see Figure 1) so that their long hydrocarbon chains are extending outwards into the lumen. This results in the formation of a uniform hydrophobic layer over the tissue, which cannot be penetrated by hydrophilic damaging agents. This prevents water soluble damaging agents (e.g., acid, microbial toxins, proteolytic enzymes) from coming in contact with the tissue, and protects the tissue from injury.

Although, it is expected that anionic surfactants would be repelled by the negatively charged surface lining, (and consequently would be ineffective in providing a protective barrier), the inclusion of anionic surfactants to the compositions of this invention is one of the preferred embodiment. One possible explanation may lie in the finding that the addition of anionic phospholipids to the suspension of amphoteric phospholipids accelerates the precipitation of these surfactants from the suspension. Thus, phospholipid suspensions containing anionic phospholipids may coat biological surfaces at a faster rate than in its absence.

In the practice of this invention, Applicants have found that gastric mucosa pretreated with either synthetic or natural phospholipids become virtually impermeable to acid. This property was most dramatically demonstrated *in vivo*, where it was observed that rats pretreated with phospholipid resisted severe gastric mucosal damage after direct challenge to a strong solution of hydrochloric acid. In contrast, saline treated rats suffered severe gastric erosion and hemorrhage. *In vitro* transport studies have confirmed these findings, demonstrating that acid ($H^+$) diffuses much more slowly across gastric mucosa exposed to luminal phospholipid surfactants than across untreated tissue.

In order that the invention may be more clearly understood, preferred embodiments will be further described in terms of the following examples, which should not be construed to limit the scope of the invention.

Example I

*In vitro* study of phospholipid protection against $H^+$ back diffusion

This experiment was performed in order to determine whether the phospholipids which are adsorbed onto the surface of the canine gastric mucosa effectively retard the mucosal to serosal flux of $H^+$ across the canine gastric mucosa. Accordingly, two adjacent strips of gastric mucosa from the oxyntic gland were bluntly disected from the underlying serosa of an anesthetized dog and mounted in a modified Ussing Chamber as described by Shanbour, *American Journal of Digestive Diseases* 19:367—371 (1974). Fifteen mls of oxygenated normal saline were added to the mucosal and nutrient compartments of both chambers and the tissue was continuously oxygenated throughout the course of the study.

In the initial studies 50 µg of the following phospholipids: dipalmitoyl lecithin (DPL), dipalmitoyl phosphatidyl ethanolamine (DPPE), dipalmitoyl phosphatidyl glycerol (DPPG), dipalmitoyl phosphatidyl inositol (DPPI) and sphingomyelin were added as a molecular suspension to the normal saline solution in the mucosal compartment of one of the chambers. According to initial estimates, the total concentration of phospholipid added to this system (250 µg) approximated the total amount of phospholipid adsorbed onto

a predetermined area of the gastric mucosal surface. Each of the phospholipids tested were obtained from Sigma Chemical Co., St. Louis, Missouri.

It was also determined that the phospholipid addition had no influence on the pH of the mucosal bathing solution which ranged between 6—7. The electrical output of the pH meters were coupled to an eight channel Beckman Dynograph (Type R411) and the pH changes in the serosal solution were monitored with time. Once a stable baseline was reached, a damaging concentration of HCl (900 mM) was added to the mucosal chamber. In seven experiments the pH in the serosal solution of the control chambers remained stable for a mean of 2.40 minutes before $H^+$ permeated into the serosal side of the chamber. The mean breaking point for $H^+$ permeation with respect to the phospholipid coated mucosa was extended to 6.70 minutes. Furthermore, it was noted that the rate of $H^+$ flux-overtime was not as rapid for the phospholipid-treated samples as compared to untreated controls. This is evident from the slope obtained for the studies. (Refer to Figure 2).

In a second series of experiments, the relative concentrations of the individual surfactants which were added to the mucosal compartment were varied without appreciably altering the total amount of phospholipid. In these experiments 135 µg of DPL was added together with 15 µg of each of the following phospholipids; PE, PG, PI and sphingomyelin. Additionally in these experiments the phospholipid suspensions were added to the compartments. This resulted in the formation of liposomal particles. These modifications further prolonged the breaking point to 10.0 minutes before $H^+$ flux was noted on the serosal side.

Example II

*In vivo* study of phospholipid protection

In the experiments described above, Applicants demonstrated that the treatment of the gastric mucosal surface with phospholipids *in vitro* significantly retarded the rate at which H+ diffused across mucosal epithelium from the lumen to the nutrient bathing solution. These results therefore suggest that phospholipids may protect against acid-induced gastric erosions by delaying the back diffusion of H+ from the lumen into the tissue. Applicants directly investigated this question in an acute pylorus-ligated rat preparation, in which test agents could be administered directly into the stomach by way of an esophageal cannula. Rats were fasted overnight and maintained under Nembutal anesthesia throughout the study period. The rats were divided into two groups (3 rats/grp); one group which was designated "surfactant treated" received 0.5 ml of a liposomal suspension in saline of the following phospholipids: 135 µg DPL, 15 µg of DPPE, DPPG, DPPI and Sphingomyelin; and the second group (controls) was intragastrically injected with 0.5 ml of saline. Five minutes later both groups received 1 ml of an ulcerogenic dose of 0.6 N HCl. The anesthetized animals were left undisturbed for 45 minutes, after which they were sacrificed, and the stomachs excised, opened along the greater curvature and examined. In contrast to the ulcerogenic appearance of the untreated control rats, rats pretreated with the surfactants grossly appeared to have little or no acid-induced damage.

In another series of *in vivo* experiments, the individual phospholipids: dipalmitoyl lecithin, dipalmitoyl phosphatidyl ethanolamine, and phosphatidyl choline were tested for anti-ulcer activity. The experiments were performed as described above. In addition, immediately before the rat was sacrificed, a gastric sample was withdrawn for hemoglobin analysis. The amount of hemoglobin present in a sample was directly proportional to the extent of GI bleeding and hence ulcerative activity. Sample anti-ulcer activity was measured as a percent of hemoglobin count relative to hemoglobin detected from animals pretreated with the combination of 135 µg DPL, 15 µg of DPPE, DPPG, DPPI and sphingomyelin mixture.

Anti-ulcer activity was inversely proportional to the amount of hemoglobin detected. Table I tabulates the relative anti-ulcer activity of the individually tested phospholipids.

TABLE I

| Phospholipid | Activity |
|---|---|
| Mixture (135 µg DPL, 15 µg each of DPPE, DPPG, DPPI and Sph.) | 100% |
| Dipalmitoyl lecithin | |
| 15 µg | 95% |
| 135 µg | 90% |
| Dipalmitoyl phosphatidyl ethanolamine | |
| 15 µg | 75—80% |
| 135 µg | 70% |
| Phosphatidyl choline | |
| 15 µg | 80% |
| 135 µg | 80% |

The foregoing description of the invention has been directed to particular embodiments for purposes of explanation and illustration. The amounts of phospholipids administered to human subjects can vary with respect to age, weight, and intensity of the ulcerative condition. The ability to determine an

appropriate and effective dosage limit consistent with the teachings of this disclosure will be apparent to those skilled in the art. Moreover, various modifications in acceptable routes of administration will be apparent to those skilled in the art when faced with a particular situation. For example, treatment of necrotizing enterocolitis in infants may be facilitated by supplementing the infant's artificial formula with synthetic phospholipid surfactants.

**Claims**

1. The use of amphoteric phospholipid surfactant(s) for the manufacture of a medicament, in a form suitable for internal administration for treating gastro-intestinal ulcer disease.
2. The use according to Claim 1, wherein the surfactant(s) is/are in a form suitable for peroral administration.
3. The use according to Claim 1 or 2, wherein the amphoteric phospholipid surfactant(s) is/are selected from lecithins, cephalins, sphingomyelins, phosphatidyl serines or combinations thereof.
4. The use according to Claim 1 or 2, wherein the amphoteric phospholipid surfactant(s) is/are selected from phosphatidyl ethanolamine, phosphatidyl monomethyl-ethanolamine, phosphatidyl dimethylethanolamine, phosphatidyl choline, phosphatidyl serines or mixtures thereof.
5. The use according to Claim 4, wherein the phosphatidyl compounds are dipalmitoyl substituted.
6. A composition for treating gastro-intestinal ulcer disease comprising amphoteric phospholipid surfactants in a form suitable for internal administration, wherein the surfactant is a mixture of phosphatidylcholine, phosphatidylethanolamine, phosphatidylserine and sphingomyelin.
7. A composition according to Claim 6, wherein an anionic phospholipid surfactant is in combination with the amphoteric phospholipid surfactant.
8. A composition according to Claim 6, wherein phosphatidyl glycerol and/or phosphatidyl inositol is in combination with the amphoteric phospholipid surfactant.

**Patentansprüche**

1. Verwendung von amphoterem(n) oberflächenaktiven Phospholipid(en) zur Herstellung eines Medikaments, in einer zur innerlichen Applikation geeigneten Form, zur Behandlung einer gastro-intestinalen Geschwürkrankung.
2. Verwendung nach Anspruch 1, dadurch gekennzeichnet, daß der (die) oberflächenaktive(n) Stoff(e) in einer zur peroralen Applikation geeigneten Form vorliegt(en).
3. Verwendung nach Anspruch 1 oder 2, dadurch gekennzeichnet, daß das (die) amphotere(n) oberflächenaktive(n) Phospholipid(e) aus Lecithinen, Cephalinen, Sphingomyelinen, Phosphatidylserinen oder Kombinationen derselben ausgewählt ist (sind).
4. Verwendung nach Anspruch 1 oder 2, dadurch gekennzeichnet, daß das (die) amphotere(n) oberflächenaktive(n) Phospholipid(e) aus Phosphatidylethanolamin, Phosphatidylmonomethylethanolamin, Phosphatidyldimethylethanolamin, Phosphatidylcholin, Phosphatidylserinen oder Mischungen derselben ausgewählt ist (sind).
5. Verwendung nach Anspruch 4, dadurch gekennzeichnet, daß die Phosphatidylverbindungen zweifach mit dem Palmitoylrest substituiert sind.
6. Zusammensetzung zur Behandlung einer gastro-intestinalen Geschwürerkrankung, gekennzeichnet durch amphoteres (amphotere) oberflächenaktives (oberflächenaktive) Phospholipid(e) in einer zur innerlichen Applikation geeigneten Form, wobei der oberflächenaktive Stoff ein Gemisch aus Phosphatidylcholin, Phosphatidylethanolamin, Phosphatidylserin und Sphingomyelin ist.
7. Zusammensetzung nach Anspruch 6, dadurch gekennzeichnet, daß ein anionisches oberflächenaktives Phospholipid in Kombination mit dem amphoteren oberflächenaktiven Phospholipid vorliegt.
8. Zusammensetzung nach Anspruch 6, dadurch gekennzeichnet, daß Phosphatidylglycerin und/oder Phosphatidylinosit in Kombination mit dem amphoteren oberflächenaktiven Phospholipid vorliegt.

**Revendications**

1. Utilisation d'un ou plusieurs surfactants phospholipidiques amphotères pour la production d'un médicament, sous une forme convenable pour l'administration par voie interne pour le traitement de l'ulcère gastrointestinal.
2. Utilisation suivant la revendication 1, dans laquelle le ou les surfactants sont sous une forme convenable pour l'administration par voie orale.
3. Utilisation suivant la revendication 1 ou 2, dans laquelle le ou les surfactants phospholipidiques amphotères sont choisis entre des lécithines, des céphalines, des sphingomyélines, des phosphatidylsérines ou leurs mélanges.
4. Utilisation suivant la revendication 1 ou 2, dans laquelle le ou les surfactants phospholipidiques amphotères sont choisis entre la phosphatidyléthanolamine, la phosphatidylmonométhyléthanolamine, la phosphatidyldiméthyléthanolamine, la phosphatidylcholine, les phosphatidylsérines ou leurs mélanges.

5. Utilisation suivant la revendication 4, dans laquelle les composés de phosphatidyle portent un substituant dipalmitoyle.

6. Composition pour le traitement de l'ulcère gastro-intestinal, comprenant un ou plusieurs surfactants phospholipidiques amphotères sous une forme convenable pour l'administration par voie interne, dans laquelle le surfactant est un mélange de phosphatidylcholine, de phosphatidyléthanolamine, de phosphatidylsérine et de sphingomyéline.

7. Composition suivant la revendication 6, dans laquelle un surfactant phospholipidique anionique est associé au surfactant phospholipidique amphotère.

8. Composition suivant la revendication 6, dans laquelle le phosphatidylglycérol et/ou le phosphatidylinositol est associé au surfactant phospholipidique amphotère.

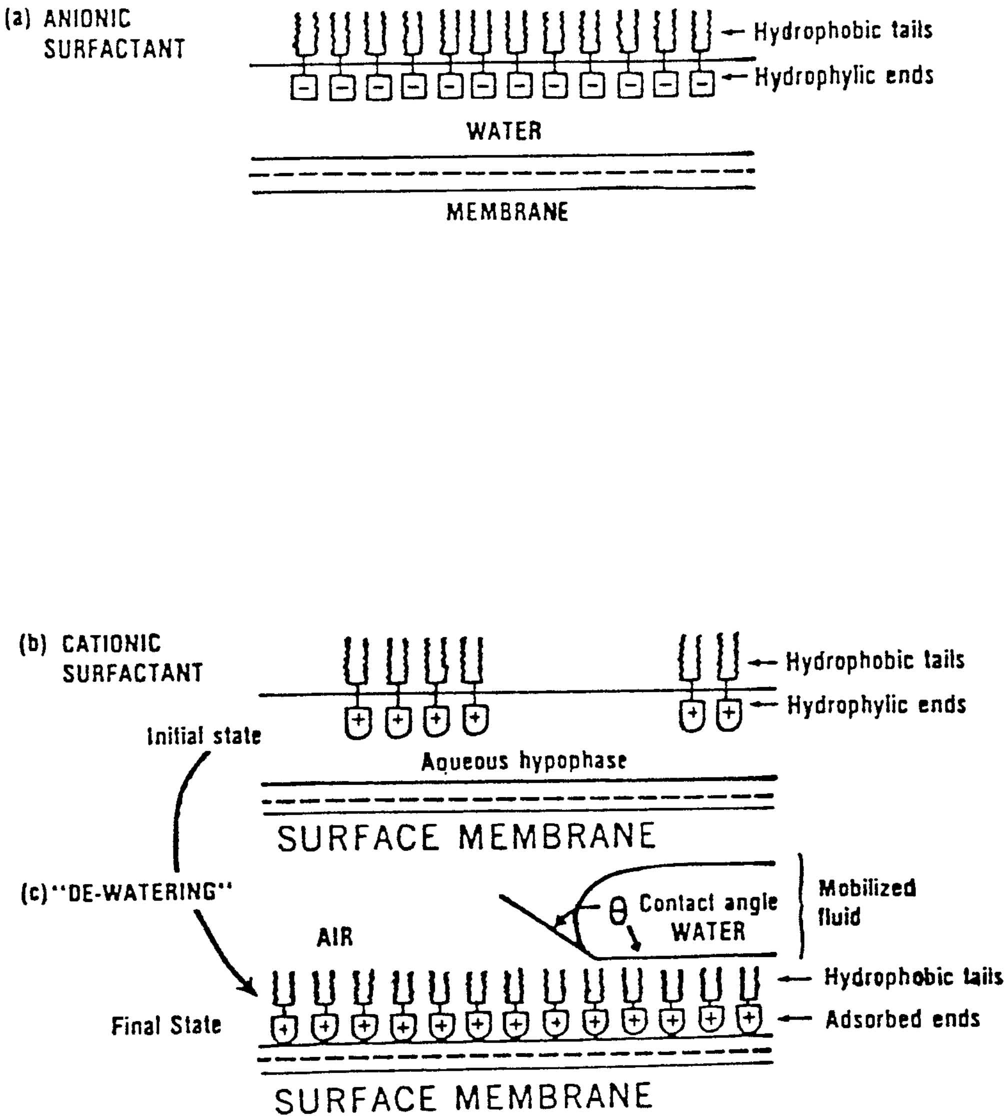
(a) ANIONIC SURFACTANT
Hydrophobic tails
Hydrophylic ends
WATER
MEMBRANE
(b) CATIONIC SURFACTANT
Hydrophobic tails
Hydrophylic ends
Initial state
Aqueous hypophase
SURFACE MEMBRANE
(c) "DE-WATERING"
Mobilized fluid
θ Contact angle
WATER
AIR
Hydrophobic tails
Adsorbed ends
Final State
SURFACE MEMBRANE

FIG. 1

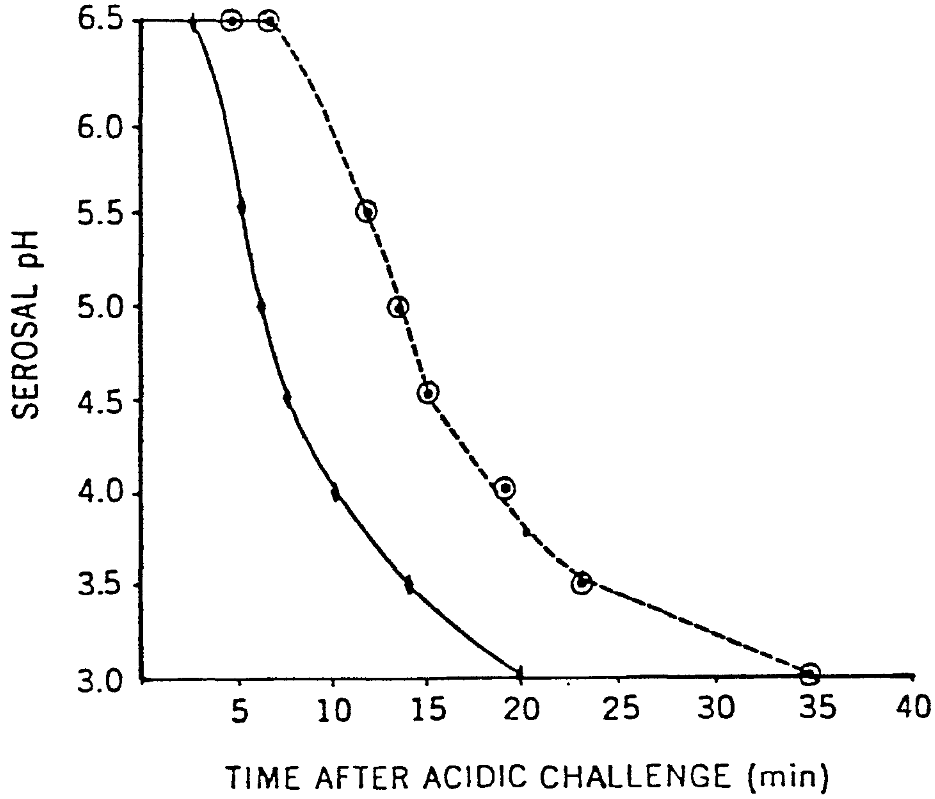
6.5
6.0
5.5
5.0
4.5
4.0
3.5
3.0
SEROSAL pH
5
10
15
20
25
30
35
40
TIME AFTER ACIDIC CHALLENGE (min)

FIG. 2